# EUROPÄISCHE PATENTSCHRIFT

(11) **EP 1 527 751 B1**
(45) Veröffentlichungstag und Bekanntmachung des Hinweises auf die Patenterteilung: **13.07.2011**
(21) Anmeldenummer: 04025476.5
(22) Anmeldetag: 27.10.2004
(51) Int. Cl.: A61F 2/06

(54) **Geflochtenes rohrförmiges Implantat**
braided tubular implant
implant tubulaire tressé

(30) Priorität: 28.10.2003 DE 10351220
(43) Veröffentlichungstag der Anmeldung: 04.05.2005
(73) Patentinhaber: Deutsche Institute für Textil- und Faserforschung Denkendorf Stiftung des öffentlichen Rechts, 73770 Denkendorf (DE)
(72) Erfinder: Hierlemann, Helmut, 73033 Göppingen (DE); Baumann, Maria, 73035 Göppingen (DE); Milwich, Dr.-Ing. Markus, 72644 Oberboihingen (DE); Planck, Prof.Dr.-Ing. Heinrich, 72622 Nürtingen (DE)
(74) Vertreter: Patentanwälte Ruff, Wilhelm, Beier, Dauster & Partner

(56) Entgegenhaltungen:
- EP-A- 0 857 471
- US-A- 5 540 713
- US-A- 5 968 088
- US-A- 6 007 574
- US-B1- 6 632 241

## Beschreibung

Zur Behandlung von Störungen an Hohlräumen im lebenden Organismus werden tubenförmige Hohlkörper, sogenannte Stents, als Endoprothesen implantiert. Sie dienen der schienenartigen Verstärkung oder Unterstützung von Hohlkörpern bei Menschen oder Tieren. Typische Anwendungsbereiche sind zum Beispiel das vaskuläre System, das gastrointestinale System und das urethrale System. Üblicherweise werden Stents in komprimierter Form mit Hilfe eines Katheters durch den zu behandelnden Hohlkörper bis and den gewünschten Behandlungsort gebracht und dort freigesetzt. Die Entfaltung des im Katheter komprimierten Stents erfolgt durch eigene Federrückstellkräfte aufgrund des Stentkonstruktionsprinzips oder mittels Ballondilatation. Es ist erforderlich, dass die Stents über lange Zeit dynamische und statische Verformungen mitmachen können, ohne nennenswerten Verlust ihrer ursprünglichen Rückstellkräfte zu erfahren. Im Idealfall ist gefordert, dass der Stent sich bezüglich des Lumens und bezüglich der Flexibilität dem Einsatzort anpasst und dauerhaft als Implantat im Körper verbleibt.

Es wurden zahlreiche Stents entwickelt, die aus metallischen Werkstoffen, synthetischen Werkstoffen, im Körper resorbierbarem oder nicht resorbierbarem Material, sowie einer Kombination von Materialien, zum Beispiel in Form einer Beschichtung hergestellt sind.

Die US-Patente Nr. 4,655,771; 4,768,507 und 4,907,336 beschreiben selbstexpandierbare, nicht resorbierbare Stents. US-Patent Nr. 4,990, 155 offenbart einen thermoreversiblen, nicht resorbierbaren Stent. In EP 0335341 und US-Patent Nr. 4,799,479 sind ballondilatierbare, nicht resorbierbare Stents beschrieben. US-Patente Nr. 4,950,258 und 5,670,161 sowie EP 0809981 betreffen thermoreversible, resorbierbare Stents. In den US-Patenten Nr. 5,980,564; 5,968,092; 5,500,013; 5,762,625; 6,080,177; 5,306,286; 4,057,537 und dem Canadischen Patent Nr. 2,025,625 sowie in EP 0,797,963 sind selbstexpandierbare, resorbierbare Stents beschrieben.

Ein rohrförmiges Implantat, insbesondere Stent, in Form eines Rundgeflechtes aus in gegenläufigen Wendeln sich überkreuzenden Fäden, wobei an den Rohrenden des Implantats liegende Fadenbereiche frei von Fadenenden sind und dort vorhandene Fäden in die Geflechtstruktur zurückgeführt sind, ist aus der US 6,632,241 B1 bekannt. Ein weiterer, gattungsgemäßer Stent ist aus der US 6,007,574 bekannt. Ein Stent zum Expandieren eines Körperlumens wird in der EP 0857471 A2 beschrieben. In der US 5,540,713 wird eine Vorrichtung zum Aufweiten einer Stenose in einer Körperröhre beschrieben. Die Vorrichtung basiert auf einer Legierung mit Formerinnerungsvermögen und einer im wesentlichen zylindermantelförmigen Außenkontur. Die US 5,968,088 betrifft einen expandierbaren Stent mit einem flexiblen röhrenförmigen Korpus.

Die derzeit im Handel erhältlichen Stents zeigen im klinischen Einsatz immer wieder ungünstige Eigenschaftsprofile und unerwünschte klinische Ergebnisse wie zum Beispiel Materialermüdung, Stentdislokation, Entzündungen, Thrombosen oder Restenosen. Diese Nachteile beeinträchtigen den Behandlungserfolg sowie die Nachhaltigkeit einer Behandlung zu Ungunsten des Patienten.

Es stellt sich daher die Aufgabe einen verbesserten Stent zur Verfügung zu stellen, der die Unzulänglichkeiten von Stents aus dem Stand der Technik überwindet, einfach und sicher einzusetzen ist.

Diese Aufgabe wird gelöst durch ein rohrförmiges Implantat, insbesondere einen Stent, gemäß Anspruch 1.

Im Gegensatz zu bekannten Stents, die aus langen Röhren oder Schläuchen abgeschnitten sind und deshalb an den Rohrenden störende Fadenenden besitzen, sind solche Fadenenden an den Rohrenden des erfindungsgemäßen Stents nicht vorhanden. Es ist deshalb auch nicht erforderlich solche Fadenenden abzudecken oder in ein anderes Material einzubinden. Unter Geflecht sind vorzugsweise diagonal verlaufende sich über- und unterkreuzende Fäden zu verstehen.

Auf diese Weise werden die Nachteile herkömmlicher nach einer Flechttechnologie hergestellter Stents überwunden, die aus einer Vielzahl von monofilen oder multifilen Fäden oder Drähten gebildet sind, bei denen nach dem Herstellungsvorgang zahlreiche stumpfe oder scharfe Schnittstellen und Schnittkanten der offenen Fadenenden auftreten, die eine Nachbehandlung durch Beschichten, Verlöten, Verschweißen oder Kaschieren erfordern, um ihre traumatisierende Wirkung zu verhindern.

Gemäß der Erfindung kann sich das rohrförmige Implantat dadurch auszeichnen, dass es eine tubuläre, radial kompressionsfähige und expandierbare sowie axial flexible Struktur aufweist. Im entlasteten Zustand, d. h. ohne Einwirkung externer radialer Kräfte, weist der Stent eine radial gleichmäßige, tubenartige Form auf. Bevorzugt kann das Implantat in radialer und axialer Richtung flexibel sein.

Mit Vorteil kann das erfindungsgemäße Implantat aus Fäden gebildet sein, die monofile Drähte sind. Die monofilen Drähte (Monofilamente) können einen Durchmesser von 30 µm bis 2 mm, insbesondere 70 µm bis 500 µm besitzen. In Weiterbildung können parallele Drähte geringfügig miteinander verdreht sein.

In einer besonderen Ausführungsform der Erfindung ist das Geflecht des Implantats aus einem einzigen Faden, das heißt einem sogenannten Endlosfaden, gebildet. Ein aus einem, insbesondere einzigen, Monofilament gebildeter selbstexpandierbarer Stent weist eine netzartige Geflechtstruktur auf.

In einer weiteren besonderen Ausführungsform der Erfindung kann das Geflecht des Implantats aus zwei parallelen vorzugsweise gegenläufigen Monofilamenten (Doppelstrang) gebildet sein und zwar vorzugsweise auch bei Ausbildung des Geflechts aus einem einzigen Endlosfaden.

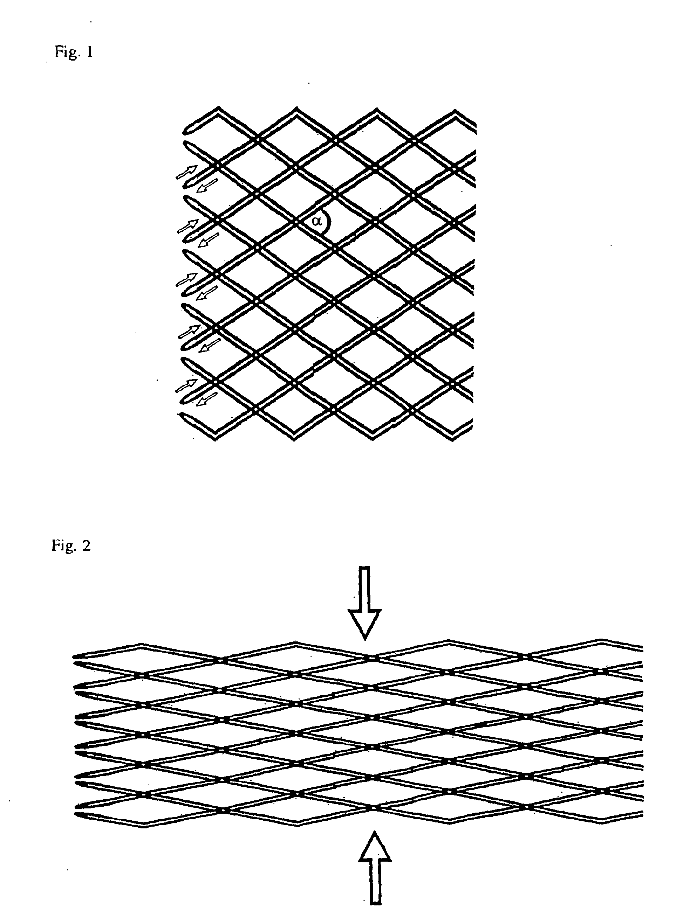

Der Fadenkreuzungswinkel α (vgl. Figur 1) im Geflecht zwischen sich kreuzenden Monofilamenten kann größer sein als 45 °, insbesondere 70 bis 150°, und bevorzugt 90 bis 120° betragen. Erfindungsgemäß können an den Implantatenden die Filamente gebogen, insbesondere kurvenförmig oder serpentinenartig ausgeformt sein. Bevorzugt können Fadenenden, insbesondere die beiden Enden des einzigen Fadens, in der Mantelebene bzw. Mantelfläche des Rundgeflechts liegen. Ferner können Fadenenden, insbesondere sämtliche Fadenenden, in der jeweiligen Wendel nahe beieinander liegen und vorzugsweise in entgegengesetzte Richtung weisen.

Es kann vorteilhaft sein, dass die Fadenbereiche an mindestens einem Rohrende umgebogen sind und in der Geflechtsebene wendelförmig zurückgeführt sind. Die Fadenbereiche können mindestens an einem Rohrende, insbesondere an einem Rohrende unter Bildung einer Kehrtwendung schlaufenartig umgebogen und in der gleichen Wendel zurückgeführt sein. Die Fadenbereiche sind an mindestens einem Rohrende, insbesondere an einem Rohrende in einem Winkel von 60 bis 120°, insbesondere um ca. 90° und in einer gegenläufigen Wendel zurückgeführt. Die Fadenbereiche sind an mindestens einem Rohrende, insbesondere an einem Rohrende unter Bildung einer sich überkreuzenden Schlaufe um 150 bis 300°, insbesondere ca. 270° umgebogen und in einer gegenläufigen Wendel zurückgeführt.

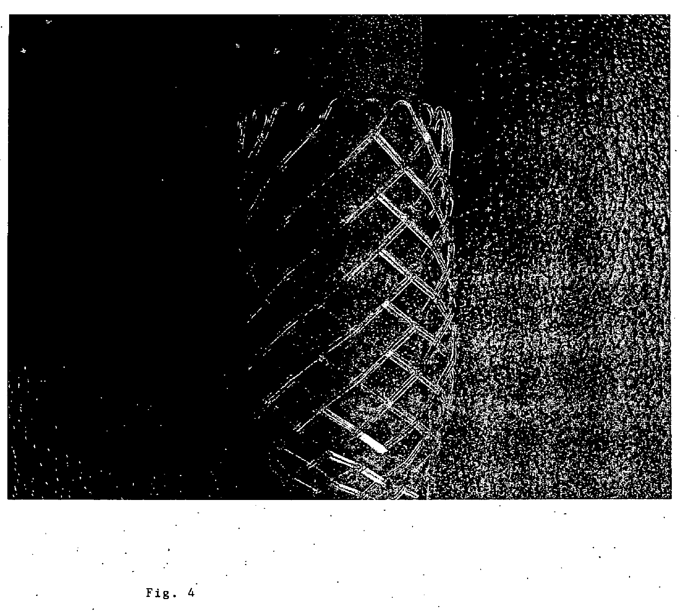

Erfindungsgemäß ist an mindestens einem Rohrende, insbesondere an einem Rohrende abwechselnd ein Fadenbereich um 60 bis 120° umgebogen und ein Fadenbereich aus derselben Wendel um 150 bis 300° als sich überkreuzende Schlaufe umgebogen und der geschlaufte Faden in der sich direkt anschließenden rückläufigen Wendel und der nur winkelförmig umgebogene Faden in der darauffolgenden parallelen rückläufigen Wendel zurückgeführt. Ein Beispiel einer solchen Ausführung ist in der begleitenden Figur 4 gezeigt.

Das erfindungsgemäße rohrförmige Implantat kann sich weiterhin dadurch auszeichnen, dass es gitterartig ausgebildet ist und im entspannten Zustand eine Gitterweite von 0,5 bis 8 mm, insbesondere 2 bis 5 mm aufweist. Dabei können die Fadenkreuzungswinkel mehr als 45°, insbesondere 70 bis 150°, bevorzugt 90 bis 120° betragen.

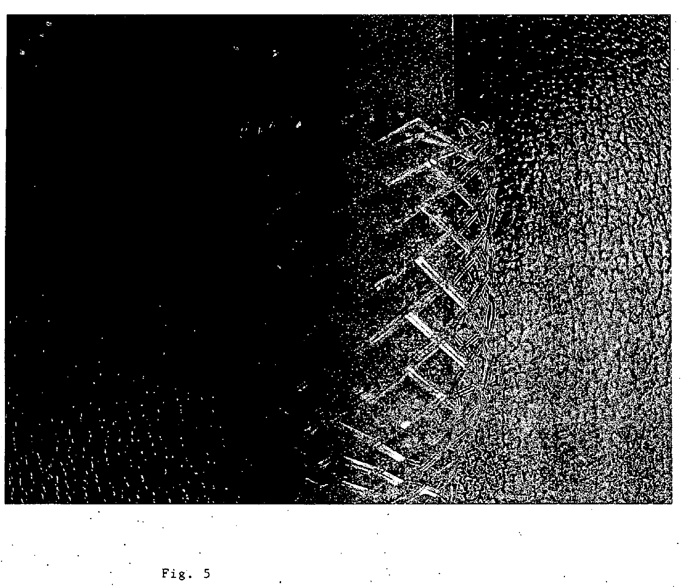

In einer bevorzugten Ausführungsform ist im Geflecht des erfindungsgemäßen Implantats jede Wendel von mindestens zwei, insbesondere zwei, parallel neben einander liegenden Fäden gebildet. Insbesondere können an einem Stentende bei geradzahliger Fadenzahl einer Wendel jeweils zwei neben einander liegende Fäden in gegenläufiger Richtung verlaufen. Ein Beispiel einer solchen Ausführung ist in der begleitenden Figur 5 gezeigt.

Gemäß der Erfindung kann in einer Ausführungsform die Geflechtstruktur einen Fadenverlauf 1 über 1, 1 unter 1 aufweisen. In einer anderen Ausführungsform kann die Geflechtstruktur einen Fadenverlauf 2 über 2, 2 unter 2 aufweisen. Mit Vorteil können in jeder Wendelrichtung bezogen auf den Querschnitt des Stents 4 bis 16, insbesondere 6 bis 12 Wendeln vorgesehen sein.

Erfindungsgemäß kann das rohrförmige Implantat mit radial gleichmäßigem Durchmesser ausgebildet sein. In einer besonderen Ausführungsform der Erfindung kann das rohrförmige Implantat endständig verjüngt sein, das heißt am Ende einen kleineren Durchmesser aufweisen. Eine solche Verjüngung des Stents kann zum Zwecke einer Filterung, z. B. im Blutstrom zweckmäßig sein. In einer anderen bevorzugten Ausführungsform der Erfindung kann das rohrförmige Implantat im entspannten Zustand endständig ausgestellt sein, das heißt an mindestens einem, vorzugsweise beiden, Enden eine größeren Durchmesser aufweisen als im Zwischenbereich. Eine solche radiale Aufdehnung kann zweckmäßig sein, um Dislokationen nach Einbringen des Stents zu verhindern.

Beim erfindungsgemäßen rohrförmigen Implantat kann mindestens eines der Implantatenden radial divergierend ausgebildet sein. Mit anderen Worten kann in einer Ausführungsform der Erfindung ein Ende des rohrförmigen Implantats aufgeweitet sein. In einer anderen Ausführungsform der Erfindung können beide Enden des rohrförmigen Implantats aufgeweitet sein. Mit Vorteil kann der Übergang vom linearen Teil des Implantats zum divergierenden Ende stufenlos sein. Eine solche Durchmessererweiterung kann trichterförmig oder tulpenförmig ausgebildet sein.

Beim Implantat gemäß der Erfindung kann das biokompatible Material metallisches Material sein. Typische Beispiele sind Metallfilamente aus Titan, Titanlegierungen, rostfreiem medizinischem Stahl, wie Cr-Ni-Stähle, W1.4310, Elgiloy®, Phynox®, Iridium oder Metalloxidlegierungen. Weiterhin kommen sogenannte Shape-Memory-Metalle wie z.B. Nitinol® in Frage.

In einer anderen Ausführungsform der Erfindung kann das biokompatible Material synthetisches Polymermaterial sein. Typische Beispiele sind Filamente aus Synthesepolymeren wie Polyethylenterephthalat (PET), Polyurethan (PUR), Polypropylen (PP), hoch-dichtes Polyethylen (HDPE), Polyamid, Copolymere, Blends oder Mischungen solcher Polymere. Für resorbierbare Implantate oder resorbierbare Teile der Implantate können bevorzugt Polymere auf Basis von α-Polyhydroxycarbonsäuren, β-Polyhydroxycarbonsäure oder Polyanhydriden in Form ihrer Homopolymere, Copolymere, Terpolymere, Blockpolymere oder Mischungen davon eingesetzt werden.

In einer besonderen Ausführungsform der Erfindung kann das biokompatible Material eine Zusammensetzung aus verschiedenen Materialien, insbesondere ein Verbundstoff sein. Typische Beispiele sind Blendpolymere, Bikomponent-Monofile wie Monofile mit Kern-Mantel-Struktur, Metall-Polymerverbundstoffe, insbesondere mit Metallmatrix sowie auch mit Polymer beschichtete Metalle. Das Fadenmaterial der Stents kann eine Oberflächenbeschichtung aus Metall aufweisen, insbesondere wenn das Fadenmaterial ein Polymer ist.

Es können zahlreiche Modifikationen von Filamenten eingesetzt werden, wie es für den gewünschten Anwendungszweck geeignet ist. Zum Beispiel strukturierte Monofile, Hohlkapillar-Monofile, beschichtete Monofile mit einlagiger oder mehrlagiger Beschichtung. So können die monofilen Drähte einen strukturierten Querschnitt aufweisen, z.B. einen sternförmigen Querschnitt oder einen Querschnitt mit Kern-Mantel-Struktur.

Das erfindungsgemäß verwendete Filamentmaterial kann in einem weiten Bereich von Faserstärken und Faserdicken (Filamentdurchmessern) vorliegen. Bevorzugt sind Durchmesser von 10 - 800 µm, insbesondere 30 - 300 µm, bei Metalldrähten und Durchmesser von 30 - 1000 µm, insbesondere 50 - 500 µm, bei Polymerfilamenten.

In einer Ausführungsform der Erfindung kann das biokompatible Material nicht bioresorbierbar sein. In einer anderen Ausführungsform der Erfindung kann das biokompatible Material mindestens teilweise bioresorbierbar sein. In noch einer anderen Ausführungsform der Erfindung kann das biokompatible Material vollständig bioresorbierbar sein.

Mit Vorteil können die Monofilamente zur Ausbildung der Geflechtstruktur des rohrförmigen Implantats eine hohe Zugfestigkeit im Bereich über 100 N/mm² und/oder einen hohen Elastizitäts-Modul im Bereich über 500 N/mm² aufweisen.

Das rohrförmige Implantat der Erfindung kann sich vorteilhaft dadurch auszeichnen, dass es elastisch und/oder plastisch ist. Die elastischen und/oder plastischen Eigenschaften beruhen auf der erfindungsgemäßen Kombination von Monofilament und Geflechtstruktur.

In Weiterbildung kann beim rohrförmigen Implantat die ursprünglich offenporige Geflechtstruktur mindestens teilweise auf der Innenseite und/oder der Außenseite von einem Überzug bedeckt sein. In einer anderen Ausführungsform kann die ursprünglich offenporige Geflechtstruktur mindestens teilweise auf der Innenseite und/oder der Außenseite von einer Beschichtung bedeckt sein. Als Beschichtung können mit Vorteil Materialien mit elastischen und/oder plastischen Eigenschaften eingesetzt sein.

Eine Beschichtung kann das erfindungsgemäße Implantat vollständig einbetten. Alternativ können nur bestimmte Teile des Implantats mit einer Beschichtung versehen sein, zum Beispiel eines oder beide Enden. Die Beschichtung kann nur das Fadenmaterial überdecken, so dass die rautenförmigen Öffnungen des Geflechts unbedeckt sind. Die Beschichtung kann insbesondere bei elastischem Beschichtungsmaterial auch die Implantatwandung verschließen. Die Beschichtung kann in Form eines sogenannten Covering ausgeführt sein, wobei das rohrförmige Implantat auf eine schon vorgebildete Hülle oder einen Film aufgezogen wird und damit innen und/oder außen überzogen wird. In einer anderen Verfahrensweise kann eine Beschichtung als sogenanntes Coating vorgenommen werden, wobei Strukturelemente des rohrförmigen Implantats mit einem Beschichtungsmaterial eine innige physikalische und/oder chemische Verbindung eingehen. Das Beschichtungsmaterial kann resorbierbar sein.

Gemäß einer Ausführungsform der Erfindung kann der Überzug und/oder die Beschichtung adhäsiv gebunden sein. Gemäß einer anderen Ausführungsform der Erfindung kann der Überzug und/oder die Beschichtung kovalent gebunden sein.

Mit Vorteil kann das erfindungsgemäße Implantat in Weiterbildung mit mindestens einem Zusatzstoff versehen sein. Insbesondere kann der Zusatzstoff ein pharmakologischer Wirkstoff sein. Als Beispiele für solche Zusatzstoffe sind Mittel zur Verbesserung der Antitrombogenität wie Hirudin, Prostacyclin, Heparin zu nennen. Bei Verwendung des erfindungsgemäßen Implantats als Drug Delivery Carrier zur Wirkstofffreisetzung können Zusatzstoffe wie Antikrebsmittel, beispielsweise Taxol®, Thalodmide® zugefügt sein. In einer anderen Ausführungsform kann der Zusatzstoff ein Röntgenmarker sein. Für ein Drug Delivery kann insbesondere eine Beschichtung bzw. ein Überzug des Fadenmaterials ausgebildet sein.

In einer speziellen Ausführungsform kann der Zusatzstoff lebende Zellen sein.

Mit Vorteil können im erfindungsgemäßen Implantat Zusatzstoffe mit Hilfe von Beschichtungstechnologien eingebracht werden. Es ist entsprechend der Wahl der Wirkstoffe und des Beschichtungsverfahrens möglich, Zusatzstoffe oberflächlich zu dotieren und/oder sie in die Polymermatrix einzulagern. Auf diese Weise kann eine Freisetzung des einen oder mehrerer zugesetzter Wirkstoffe über das Degradationsverhalten und/oder das Resorptionsverhalten des verwendeten Polymermaterial gesteuert werden.

Gegenstand der Beschreibung ist auch ein Verfahren zur Herstellung eines rohrförmigen Implantats aus biokompatiblem Material in Monofilamentform durch textile Flechtverfahren zur Ausbildung eines flexiblen, röhrenförmigen Geflechts mit endständig geschlossener Struktur. Zur Ausbildung des rohrförmigen Implantats gemäß der Erfindung kann das Flechten mit Vorteil über einem Dorn vorgenommen werden. In bevorzugter Weiterbildung ist das Flechten maschinell, insbesondere automatisch vorgenommen. Das rohe Geflecht kann einer Nachformung, thermischen Nachbehandlung (Tempern), Beschichtung, Covering, Coating oder einer beliebigen Kombination solcher Bearbeitungen unterzogen werden. Bevorzugt kann das rohe Geflecht für das rohrförmige Implantat thermisch nachbehandelt werden.

Der Vorteil des erfindungsgemäßen Herstellverfahrens ist, dass eine distal und proximal geschlossene, atraumatische Geflechtkonstruktion ausgebildet wird. Auf diese Weise ist ein Kaschieren der Stentenden sowie ähnliche Nachbearbeitungsschritte unnötig.

Mit Vorteil ist ein rohrförmiges Implantat gemäß der Erfindung geeignet zur Verwendung bei der Behandlung von pathologisch veränderten Defektstellen an Hohlorganen in der Humanmedizin und Veterinärmedizin. Als Beispiele sind zu nennen: maligne und benigne Obstruktionen, Stenosen, Ausstülpungen (Aneurysmen) und Läsionen an Hohlorganen. Typische Einsatzgebiete für erfindungsgemäße Stents sind Blutgefäße, Oesophagus, Trachea, Duodenum, Colon und andere Teile des Verdauungssystems sowie Harnwege und Harnleiter. Mit besonderem Vorteil kann das rohrförmige Implantat gemäß der Erfindung Verwendung finden an Hohlorganen im vaskulären, gastrointestinalen, tracheo-bronchialen und/oder urethralen Bereich.

Das erfindungsgemäße rohrförmige Implantat ist geeignet, ein menschliches oder tierisches Hohlorgan über ein bestimmtes Zeitintervall oder auf Dauer zu stützen und/oder offen zu halten. Dieses Zeitintervall ist abhängig vom gewählten Material und kann je nach medizinischer Anforderung genau angepasst werden. Auch können mit dem erfindungsgemäßen Implantat mechanische und physiologische Anforderungen wie Durchmesser, Rückstellkraft, Kompressionskraft und Flexibilität sehr genau eingestellt werden.

Zur praktischen Anwendung kann das rohrförmige Implantat gemäß der Erfindung mit handelsüblichen Kathetern komprimiert, an den Behandlungsort gebracht und mit üblichen Freisetzungssystemen in situ platziert werden. Das erfindungsgemäße rohrförmige Implantat ist aufgrund seiner Struktur selbstexpandierend und wird mit einer entsprechend gewählten Rückstellkraft gegen das zu behandelnde Hohlorgan gedrückt.

Im Folgenden wird die vorliegende Erfindung durch Beschreibung besonderer Ausführungsformen anhand von Beispielen und mit Bezug zu den begleitenden Zeichnungen erläutert. In diesen Ausführungsformen können Einzelmerkmale der Erfindung allein oder in Kombination mit anderen Merkmalen verwirklicht sein. Eine beschriebene besondere Ausführungsform dient lediglich zur Erläuterung und zum besseren Verständnis der Erfindung und ist in keinster Weise als Einschränkung zu verstehen.

### Kurze Beschreibung der Figuren

Fig. 1 zeigt einen Endabschnitt eines unkomprimierten rohrförmigen Stents. Die Pfeile geben Verschiebungsmöglichkeiten der Monofilamente in der Geflechtstruktur des erfindungsgemäßen rohrförmigen Implantats an. Mit α ist der Fadenkreuzungswinkel im Geflecht bezeichnet. Am Stentende sind die Monofile umgebogen und in der gleichen Wendel zurückgeführt.

Fig. 2 zeigt den Stent und nach Figur 1 unter radialer Kompression und axialer Dilatation. Die Pfeile geben die Einwirkung der Kompressionskraft an.

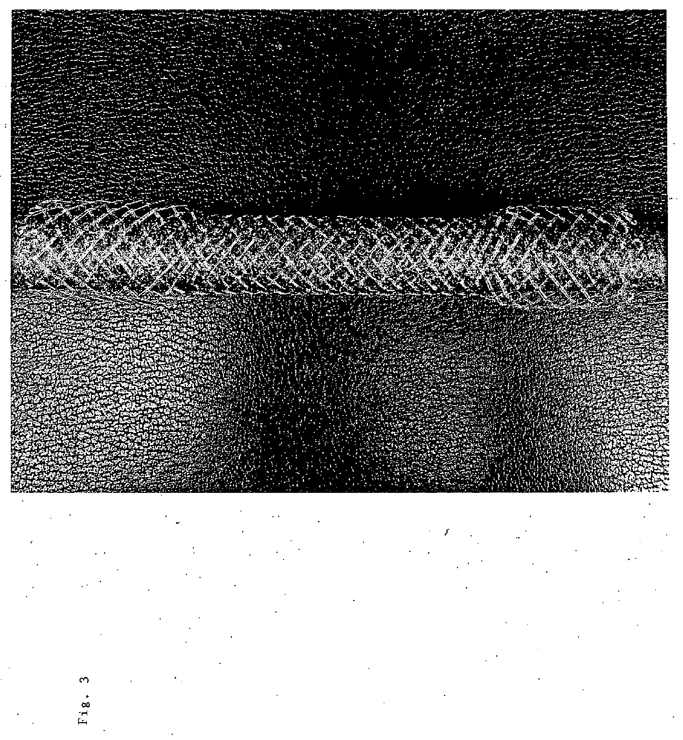

Fig. 3 zeigt einen vollständig ausgebildeten rohrförmigen Stent mit beidseitig divergierenden Enden, das heißt mit tulpenförmigen Erweiterungen am distalen und proximalen Ende. Diese Ausführungsform ist in Beispiel 1 beschrieben. Die beiden Rohrenden zeigen eine verschiedene Rückführung der Fäden in der Mantelebene des Rohrgeflechts. Das Geflecht besteht aus einem einzigen monofilen Faden. Die beiden nicht zu sehenden Fadenenden, die miteinander verbunden sein können, liegen in der Mantelfläche des Geflechts.

Fig. 4 zeigt in einer Ausführungsform den Filamentverlauf am Ende des Geflechtstents, insbesondere an einem (oberen) Ende des Stents nach Figur 3. Es sind deutlich die Verschlaufungen zu sehen, so dass keine freien Fadenenden am Ende abstehen. Am Rohrende wechseln sich abgewinkelte und überkreuzt verschlaufte Fadenbereiche ab.

Fig. 5 zeigt in einer anderen Ausführungsform den Filamentverlauf am Ende des Geflechtstents, insbesondere an einem anderen (unteren) Ende des Stents nach Figur 3. Auch hier sind schlingenförmige Fadenschlaufen zu sehen, so dass keine freien Fadenenden am Ende abstehen.

Bei allen Figuren sind je zwei Monofilamente paarweise in den Wendeln des Geflechts geführt, was bevorzugt ist.

### Beispiel 1

### Stent für den Bereich Ösophagus/Trachea

Das verwendete Fadenmaterial ist ein Polyestermonofilament aus Polyethylenterephthalat (PET) mit einem Filamentdurchmesser von 0,3 mm. Das Geflecht wurde mit einem Fadenkreuzungswinkel von 110° über einem Dorn von 18 mm Durchmesser ausgebildet. Die Stentenden sind beidseitig radial divergierend. Der Durchmesser des Stentendes beträgt 24 mm. Siehe hierzu Fig. 3.

### Beispiel 2

### Stent für den Bereich Gallengang

Das verwendete Fadenmaterial ist ein Polylactidmonofilament aus P-L-LA mit einem Filamentdurchmesser von 0,3 mm. Das Geflecht wurde mit einem Fadenkreuzungswinkel von 100° über einem Dorn von 8 mm Durchmesser ausgebildet. Der Stent weist ein gleichbleibendes Lumen auf, das heißt, die Stentenden sind nicht divergierend ausgebildet, und weisen einen Durchmesser von 8 mm auf.

### Beispiel 3

### Stent für den Bereich Colon

Das verwendete Fadenmaterial ist ein Draht aus rostfreiem Edelstahl des Typs W 1.4310 mit einem Durchmesser von 0,15 mm. Das Geflecht wurde mit einem Fadenkreuzungswinkel von 90° über einem Dorn von 22 mm Durchmesser ausgebildet. Der Stent ist einseitig radial divergierend ausgebildet. Der Durchmesser beträgt am Stentende 28 mm.

Die Herstellung des erfindungsgemäßen Implantats, insbesondere Stents, ist durch maschinelles Flechten möglich. Bei einer bevorzugten Ausführungsform wird ein einziger, insbesondere monofiler, Faden in zueinander parallele längsgerichtete Schlaufen in rohrförmiger Anordnung gelegt. Diese Schlaufen aus je zwei parallel nebeneinanderliegenden Fäden werden gleichzeitig abwechselnd rechts und links geschlagen und untereinander verflochten, wobei sich ein Schlauch- bzw. Rohrgeflecht aus rechts- und linksgängigen Wendeln ergibt, das anschließend fixiert, insbesondere hitzefixiert wird.

## Patentansprüche

1. Rohrförmiges Implantat, insbesondere Stent, in Form eines Rundgeflechtes aus in gegenläufigen Wendeln verlaufenden sich überkreuzenden Fäden aus biokompatiblem Material, wobei an den Rohrenden liegende Fadenbereiche frei von Fadenenden sind und dort vorhandene Fäden in die Geflechtsstruktur zurückgeführt sind, **dadurch gekennzeichnet, dass** an mindestens einem Rohrende abwechselnd ein Fadenbereich um 60 bis 120° umgebogen und ein Fadenbereich aus der selben Wendel um 150 bis 300° als sich überkreuzende Schlaufe umgebogen ist und der geschlaufte Faden in der sich direkt anschließenden rückläufigen Wendel und der nur winkelförmig umgebogene Faden in der darauffolgenden parallelen rückläufigen Wendel zurückgeführt sind.

2. Rohrförmiges Implantat nach Anspruch 1, **dadurch gekennzeichnet, dass** die Fäden monofile Drähte sind.

3. Rohrförmiges Implantat nach Anspruch 2, **dadurch gekennzeichnet, dass** die monofilen Drähte einen Durchmesser von 30 µm bis 2 mm, insbesondere 70 µm bis 500 µm besitzen.

4. Rohrförmiges Implantat nach einem der vorhergehenden Ansprüche, **dadurch gekennzeichnet, dass** das Geflecht aus einem einzigen Faden, einem Endlosfaden, geflochten ist.

5. Rohrförmiges Implantat nach einem der vorhergehenden Ansprüche, **dadurch gekennzeichnet, dass** Fadenenden in der Mantelebene des Rundgeflechts liegen.

6. Rohrförmiges Implantat nach Anspruch 4, **dadurch gekennzeichnet, dass** die beiden Enden des einzigen Fadens in der Mantelebene des Rundgeflechts liegen.

7. Rohrförmiges Implantat nach einem der vorhergehenden Ansprüche, **dadurch gekennzeichnet, dass** Fadenenden, insbesondere sämtliche Fadenenden, jeweils in einer Wendel nahe beieinander liegen.

8. Rohrförmiges Implantat nach einem der vorhergehenden Ansprüche, **dadurch gekennzeichnet, dass** Fadenenden, insbesondere sämtliche Fadenenden, jeweils in einer Wendel nahe beieinander liegen und in entgegengesetzte Richtung weisen.

9. Rohrförmiges Implantat nach einem der vorhergehenden Ansprüche, **dadurch gekennzeichnet, dass** es gitterartig ausgebildet ist und im entspannten Zustand eine Gitterweite von 0,5 bis 8 mm, insbesondere 2 bis 5 mm aufweist.

10. Rohrförmiges Implantat nach einem der vorhergehenden Ansprüche, **dadurch gekennzeichnet, dass** an einem Rohrende abwechselnd ein Fadenbereich um 60 bis 120° umgebogen und ein Fadenbereich aus derselben Wendel um 150 bis 300° als sich überkreuzende Schlaufe umgebogen ist und der geschlaufte Faden in der sich direkt anschließenden rückläufigen Wendel und der nur winkelförmig umgebogene Faden in der darauffolgenden parallelen rückläufigen Wendel zurückgeführt sind.

11. Rohrförmiges Implantat nach einem der vorhergehenden Ansprüche, **dadurch gekennzeichnet, dass** jede Wendel von mindestens zwei, insbesondere zwei parallel neben einander liegenden Fäden gebildet wird.

12. Rohrförmiges Implantat nach einem der vorhergehenden Ansprüche, **dadurch gekennzeichnet, dass** bei geradzahliger Fadenzahl einer Wendel jeweils zwei neben einander liegende Fäden in gegenläufiger Richtung verlaufen.

13. Rohrförmiges Implantat nach einem der vorhergehenden Ansprüche, **dadurch gekennzeichnet, dass** die Geflechtstruktur einen Fadenverlauf 1 über 1, 1 unter 1 besitzt.

14. Rohrförmiges Implantat nach einem der Ansprüche 1 bis 12, **dadurch gekennzeichnet, dass** die Geflechtstruktur einen Fadenverlauf 2 über 2, 2 unter 2 besitzt.

15. Rohrförmiges Implantat nach einem der vorhergehenden Ansprüche, **dadurch gekennzeichnet, dass** in jeder Wendelrichtung der gegenläufigen Wendeln, bezogen auf den Querschnitt des Implantats, 4 bis 16, insbesondere 6 bis 12, Wendeln vorgesehen sind.

16. Rohrförmiges Implantat nach einem der vorhergehenden Ansprüche, **dadurch gekennzeichnet, dass** es aus schlangenlinienförmig zusammenhängenden parallel längsgerichteten rohrförmig angeordneten Schlaufen geflochten ist.

## Claims

1. Tubular implant, in particular a stent, in the form of a round braid composed of threads of biocompatible material extending in oppositely directed helices and crossing over each other, with thread areas located at the tube ends being free from thread ends, and threads present there being guided back into the braid structure, **characterized in that** at least at one tube end, alternately, one thread area is turned back at 60 to 120° and one thread area from the same helix is turned back at 150 to 300° as a cross-over loop, and the looped thread is guided back in the directly contiguous return helix, and the thread turned back only angularly is guided back in the succeeding parallel return helix.

2. Tubular implant according to Claim 1, **characterized in that** the threads are monofilaments.

3. Tubular implant according to Claim 2, **characterized in that** the monofilaments have a diameter of 30 µm to 2 mm, in particular 70 µm to 500 µm.

4. Tubular implant according to one of the preceding claims, **characterized in that** the braid is braided from a single thread, a continuous thread.

5. Tubular implant according to one of the preceding claims, **characterized in that** thread ends lie in the circumferential plane of the round braid.

6. Tubular implant according to Claim 4, **characterized in that** the two ends of the single thread lie in the circumferential plane of the round braid.

7. Tubular implant according to one of the preceding claims, **characterized in that** thread ends, in particular all of the thread ends, lie close to one another in each case in a helix.

8. Tubular implant according to one of the preceding claims, **characterized in that** thread ends, in particular all of the thread ends, lie close to one another in each case in a helix and point in opposite directions.

9. Tubular implant according to one of the preceding claims, **characterized in that** it is designed as a lattice and, in the unstressed state, has a lattice width of 0.5 to 8 mm, in particular 2 to 5 mm.

10. Tubular implant according to one of the preceding claims, **characterized in that**, at one tube end, one thread area is turned back at 60 to 120° and one thread area from the same helix is turned back at 150 to 300° as a cross-over loop, and the looped thread is guided back in the directly contiguous return helix, and the thread turned back only angularly is guided back in the succeeding parallel return helix.

11. Tubular implant according to one of the preceding claims, **characterized in that** each helix is made up of at least two threads, in particular two threads lying parallel alongside one another.

12. Tubular implant according to one of the preceding claims, **characterized in that**, when a helix has a thread count which is an even number, two threads lying alongside one another in each case extend in opposite directions.

13. Tubular implant according to one of the preceding claims, **characterized in that** the braid structure has a thread profile of 1 over 1, 1 under 1.

14. Tubular implant according to one of Claims 1 through 12, **characterized in that** the braid structure has a thread profile of 2 over 2, 2 under 2.

15. Tubular implant according to one of the preceding claims, **characterized in that**, relative to the cross section of the implant, 4 to 16, in particular 6 to 12, helices are provided in each helix direction of the oppositely directed helices.

16. Tubular implant according to one of the preceding claims, **characterized in that** it is braided from serpentine, continuous, parallel, longitudinally directed, tubularly disposed loops.

## Revendications

1. Implant tubulaire, en particulier un stent, sous forme d'une tresse ronde en brins s'étendant en hélices opposées, se croisant, en matériau biocompatible, les zones de brins se trouvant aux extrémités du tube étant exemptes d'extrémités de brins et les brins qui s'y trouvent étant ramenés dans la structure de la tresse, **caractérisé en ce qu'**en au moins une extrémité de tube, alternativement, une zone de brin est retournée de 60 à 120° et une zone de brin de la même hélice est retournée de 150 à 300° en tant que boucle qui se croise et les brins en boucle sont ramenés dans l'hélice en retour directement consécutive et les brins qui ne sont retournés que sous un angle sont ramenés dans l'hélice en retour parallèle suivante.

2. Implant tubulaire selon la revendication 1, **caractérisé en ce que** les brins sont des monofilaments.

3. Implant tubulaire selon la revendication 2, **caractérisé en ce que** les monofilaments présentent un diamètre de 30 µm à 2 mm, en particulier de 70 µm à 500 µm.

4. Implant tubulaire selon l'une quelconque des revendications précédentes, **caractérisé en ce que** la tresse est tressée en un seul brin, un brin continu.

5. Implant tubulaire selon l'une quelconque des revendications précédentes, **caractérisé en ce que** les extrémités des brins se trouvent dans le plan de l'enveloppe de la tresse ronde.

6. Implant tubulaire selon la revendication 4, **caractérisé en ce que** les deux extrémités d'un même brin se trouvent dans le plan de l'enveloppe de la tresse ronde.

7. Implant tubulaire selon l'une quelconque des revendications précédentes, **caractérisé en ce que** des extrémités des brins, en particulier toutes les extrémités des brins, se trouvent à chaque fois à proximité les unes des autres dans une hélice.

8. Implant tubulaire selon l'une quelconque des revendications précédentes, **caractérisé en ce que** des extrémités des brins, en particulier toutes les extrémités des brins se trouvent à chaque fois à proximité les unes des autres dans une hélice et pointent dans une direction opposée.

9. Implant tubulaire selon l'une quelconque des revendications précédentes, **caractérisé en ce qu'**il est réalisé en forme de grille et présente, à l'état détendu, une largeur de grille de 0,5 à 8 mm, en particulier de 2 à 5 mm.

10. Implant tubulaire selon l'une quelconque des revendications précédentes, **caractérisé en ce qu'**en une extrémité de tube, alternativement, une zone de brin est retournée de 60 à 120° et une zone de brin de la même hélice est retournée de 150 à 300° en tant que boucle qui se croise et les brins en boucle sont ramenés dans l'hélice en retour directement consécutive et les brins qui ne sont retournés que sous un angle sont ramenés dans l'hélice en retour parallèle suivante.

11. Implant tubulaire selon l'une quelconque des revendications précédentes, **caractérisé en ce que** chaque hélice est formée par au moins deux, en particulier deux, brins parallèles se trouvant l'un à côté de l'autre.

12. Implant tubulaire selon l'une quelconque des revendications précédentes, **caractérisé en ce que** dans le cas d'un nombre pair de brins d'une hélice, à chaque fois deux brins adjacents s'étendent en sens opposé.

13. Implant tubulaire selon l'une quelconque des revendications précédentes, **caractérisé en ce que** la structure de la tresse présente une allure des brins 1 sur 1, 1 sous 1.

14. Implant tubulaire selon l'une quelconque des revendications 1 à 12, **caractérisé en ce que** la structure de la tresse présente une allure des brins 2 sur 2, 2 sous 2.

15. Implant tubulaire selon l'une quelconque des revendications précédentes, **caractérisé en ce que** dans chaque sens d'hélice des hélices opposées, par rapport à la section de l'implant, 4 à 16, en particulier 6 à 12, hélices sont prévues.

16. Implant tubulaire selon l'une quelconque des revendications précédentes, **caractérisé en ce qu'**il est tressé avec des boucles en forme de serpentine, solidaires, orientées parallèlement dans le sens longitudinal, disposées de manière tubulaire.
